# EUROPEAN PATENT SPECIFICATION

(11) **EP 3 481 299 B1**
(45) Date of publication and mention of the grant of the patent: **05.05.2021**
(21) Application number: 17824691.4
(22) Date of filing: 16.06.2017
(51) Int. Cl.: A61B 5/15, A61M 5/32, A61B 5/154

(54) **BLOOD COLLECTION TUBE HOLDER WITH SLIDE-ACTIVATED NEEDLE RETRACTION**
BLUTENTNAHMERÖHRCHENHALTER MIT SCHIEBERAKTIVIERTEM NADELRÜCKZUG
SUPPORT DE TUBE DE PRÉLÈVEMENT SANGUIN À RÉTRACTION D'AIGUILLE ACTIVÉE PAR COULISSEMENT.

(30) Priority: 05.07.2016 US 201615202179
(43) Date of publication of application: 15.05.2019
(73) Proprietor: Retractable Technologies, Inc., Little Elm, TX 75608 (US); Shaw, Thomas J., Frisco, TX 75034 (US)
(72) Inventor: SHAW, Thomas, J., Frisco, TX 75034 (US); SMALL, Mark, Heavener, OK 74937 (US); ZHU, Ni, Allen, TX 75013 (US)
(74) Representative: Grünecker Patent- und Rechtsanwälte PartG mbB
(86) International application number: PCT/US2017/037839
(87) International publication number: WO 2018/009329

(56) References cited:
- WO-A1-2005/087102
- US-A- 5 052 403
- US-A- 5 085 640
- US-A1- 2003 171 695
- US-A1- 2012 071 790
- US-A1- 2014 171 876
- US-A1- 2014 276 445

## Description

### BACKGROUND OF THE INVENTION

### 1. Field of the Invention

This invention is a medical device configured for use in drawing and collecting samples of bodily fluids, and especially vascular fluids such as blood.

The invention relates to a fluid collection tube holder comprising two longitudinally separated, oppositely facing, preferably coaxially alignable, needles. One needle faces forwardly and is suitable for insertion into a patient. The other needle faces rearwardly and is suitable for insertion into a collection tube for bodily fluids that is preferably evacuated. A flash chamber is desirably but not necessarily provided between the two needles to alert the user that the forwardly facing needle tip is positioned so that a bodily fluid is flowing through the needle.

The blood collection tube holder further has slide-activated needle retraction that is selectively initiated by the concurrent application of digital pressure to opposite sides of the device, and wherein the invention comprises a sliding interface disposed between opposed and facing surfaces of a body and a frontal attachment, and transverse to the direction of fluid flow through the device.

Also disclosed is a medical device having a body, a frontal attachment disposed in transverse sliding engagement with the body, two oppositely directed, coaxially aligned needles seated in the body and in the frontal attachment, respectively, and a needle retraction chamber in the body that is offset laterally from a fluid flow path through the body. Following collection of a fluid sample, the user initiates relative sliding movement along an interface between the body and the frontal attachment that is transverse to the original fluid flow path. This movement realigns the forwardly facing needle with the needle retraction chamber so that the forwardly facing needle is retracted into the needle retraction chamber and the associated needle tip is no longer exposed, thereby lessening the likelihood of accidental needle stick injury and the possibility of contamination by fluid-borne pathogens. Needle retraction can optionally be achieved before or after withdrawing the forwardly projecting needle from the patient and before or after withdrawing a fluid collection tube (such as a Vacutainer® blood collection tube often used during venipuncture procedures) from a patient's vein.

Further, according to the invention, optional structures useful for avoiding premature lateral repositioning of the body relative to the frontal attachment are also disclosed. Such structures can include, for example and without limitation, a locking cap that engages the body or another rupturable, breakable, frangible, frictionally engageable or otherwise selectively displaceable physical barrier that restricts premature transverse lateral movement of the frontal attachment relative to the body of the device.

### 2. Description of Related Art

U.S. 5,810,775 and RE39,107 disclose Medical devices such as blood collection tube holders having a single double-ended needle that is retractable into the cylindrical body of the tube holder following use. Needle retraction is initiated by closing a hinged cap upon removal of a fluid collection tube, which causes a coaxially aligned inner sleeve to move forwardly and release a rearwardly biased needle.

U.S. 645,962 and U.S. D660,420 disclose a housing for a collection device for bodily fluids that comprises a substantially cylindrical section having a forwardly extending cylindrical nose, an open rear end, a plurality of longitudinally extending ribs disposed on each side of the cylindrical section, and an elongate arm pivotably mounted near the rear of the housing over an upwardly facing slot in the tube holder.

U.S. 8,496,600 discloses a non-reusable collection device for bodily fluids such as vascular blood, the device having a housing configured similarly to that of U.S. D645,962, wherein a single, rearwardly biased double-ended needle is constrained prior to needle retraction by a rotatably mounted lug ring. The needle is released during retraction by depressing a pivotably mounted trigger connected to the body of the device to contact and rotate the lug ring, after which the needle holder is driven into a retraction cavity disposed inside the trigger while the trigger is disposed at an angle that intersects the central longitudinal axis through the housing.

U.S. 9,247,899 discloses a blood draw device with a single, double-ended retractable needle that is similar in form and function to the device of U.S. 8,496,600 except that a retainer clip retains the rearwardly biased double-ended needle until the front portion of an actuator (similar to the trigger of the '600 patent) is pivoted downwardly to cause the retainer clip to release the needle holder, after which a compressed spring expands and drives the needle holder into a retraction cavity inside the actuator that is disposed at an angle that intersects the central longitudinal axis through the housing.

U.S. 8,469,927 discloses an actuator that moves relative to the housing but does not have a rearwardly facing needle and is not disclosed for use with a fluid collection tube insertable into the housing.

US2014/0171876 A1 discloses a configuration where a frontal attachment is moveable such that the needle can be brought in a configuration such that it can be retracted in a retraction chamber.

A blood collection needle assembly, including a venipuncture needle and a fluid discharge needle is described in US 2014/0276445 A1.

### SUMMARY OF THE INVENTION

According to the invention there is provided a blood collection tube holder comprising: a body, a rearwardly facing fluid discharge needle and a needle retraction chamber laterally spaced apart from the fluid discharge needle; wherein the blood collection tube holder comprises a frontal attachment disposed in transverse sliding relation to the body, the frontal attachment comprising a needle retraction mechanism, further comprising a forwardly projecting venipuncture needle that is coaxially alignable with the fluid discharge needle when the frontal attachment is disposed in a first position relative to the body, and that is retractable into the needle retraction chamber when the body is repositioned laterally relative to the frontal attachment so that the needle retraction chamber becomes coaxially alignable with the venipuncture needle.

### BRIEF DESCRIPTION OF THE DRAWINGS

The invention is further described and explained in relation to the following drawings wherein:
FIG. 1 is a top front perspective view of one embodiment of a medical device of the invention;
FIG. 2 is an exploded top front perspective view of the medical device of FIG. 1, also showing a locking needle cap that is not depicted in FIG. 1;
FIG. 3 is a plan view of the medical device of FIG. 1 into which a fluid collection tube has been inserted for use in obtaining a sample of a vascular fluid;
FIG. 4 is a cross-sectional plan view taken along line 4-4 of FIG. 5;
FIG. 5 is a right side elevation view of the medical device of FIG. 3;
FIG. 6 is a plan view of the medical device of FIG. 3 following transverse repositioning of the body relative to the frontal attachment and retraction of the needle, but prior to withdrawal of the fluid collection tube from the body of the subject medical device;
FIG. 7 is a cross-sectional plan view of the medical device of FIG. 6 but taken along line 7-7 of FIG. 9;
FIG. 8 is a front perspective view of the medical device of FIG. 1 following transverse repositioning of the body relative to the frontal attachment and retraction of the needle, and following withdrawal of the fluid collection tube (shown in FIGS. 3-7 and 9) from the body of the subject medical device;
FIG. 9 is a right side elevation view of the medical device as in FIG. 5 following transverse repositioning of the body relative to the frontal attachment and retraction of the needle, but prior to withdrawal of the fluid collection tube from the body of the subject medical device;
FIG. 10 is a top front perspective view of another embodiment of a medical device of the invention;
FIG. 11 is a top front perspective view of the medical device of FIG. 10 following transverse repositioning of the body relative to the frontal attachment and retraction of the needle;
FIG. 12 is an exploded top front perspective view of the medical device of FIG. 10, also showing a locking needle cap that is not depicted in FIG. 10;
FIG. 13 is a front elevation view of the medical device of FIG. 10 with the locking needle cap of FIG. 12 attached;
FIG. 14 is a cross-sectional view taken along line 14-14 of FIG. 10, but rotated 90°;
FIG. 15 is a front elevation view of the medical device of FIG. 10 following transverse repositioning of the body relative to the frontal attachment and retraction of the needle;
FIG. 16 is a cross-sectional view taken along line 16-16 of FIG. 15, but rotated 90°;
FIG. 17 is a top front perspective view of another embodiment of a medical device of the invention (with a locking needle cap installed over the forwardly projecting needle) that is shown with the body connected to a separate fluid collection tube holder;
FIG. 18 is an exploded top front perspective view of the medical device of FIG. 17 but not showing the fluid collection tube holder;
FIG. 19 is a cross-sectional plan view of the medical device of FIG. 17, again showing the body connected to a fluid collection tube holder;
FIG. 20 is the medical device of FIG. 19 following removal of the locking needle cap, transverse repositioning of the body relative to the frontal attachment and retraction of the forwardly projecting needle;
FIG. 21 is a top front perspective view of the medical device of FIG. 17;
FIG. 22 is a plan view of another embodiment of a medical device of the invention wherein a friction element is provided to reduce the likelihood of accidental transverse repositioning of the body relative to the frontal attachment;
FIG. 23 is an enlarged detail view taken from FIG. 22;
FIG. 24 is a plan view of another embodiment of a medical device of the invention wherein a breakable or severable link is provided between the body and the frontal attachment to reduce the likelihood of accidental transverse repositioning of the body relative to the frontal attachment; and
FIG. 25 is an enlarged detail view taken from FIG. 24.

### DETAILED DESCRIPTION

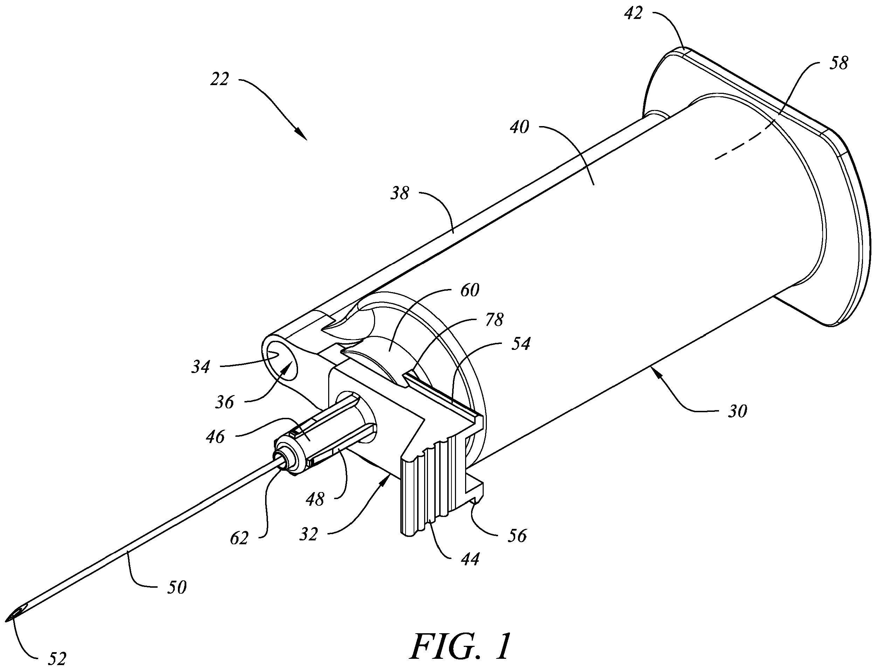

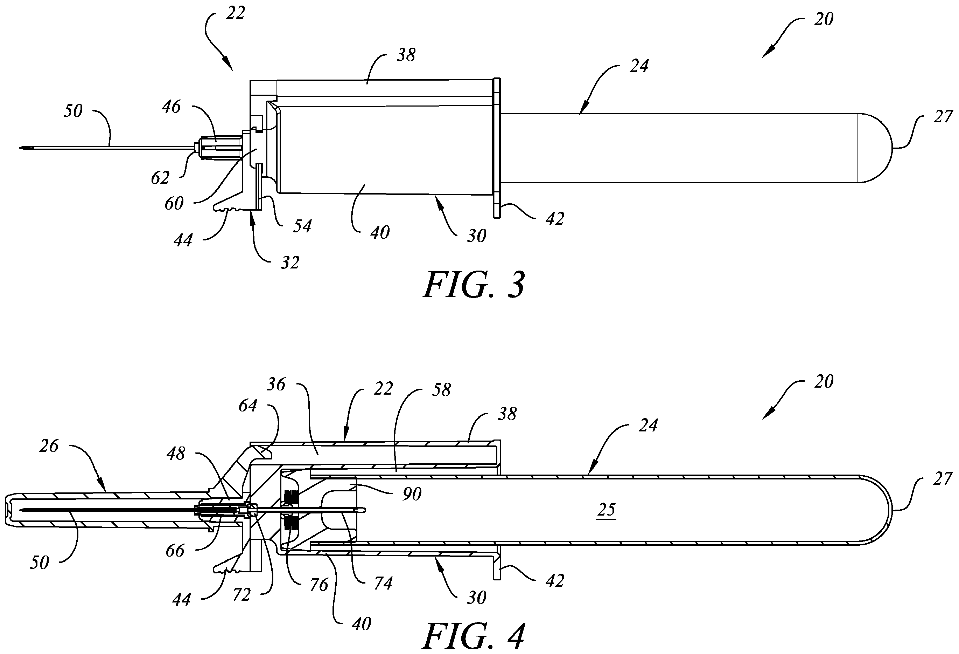

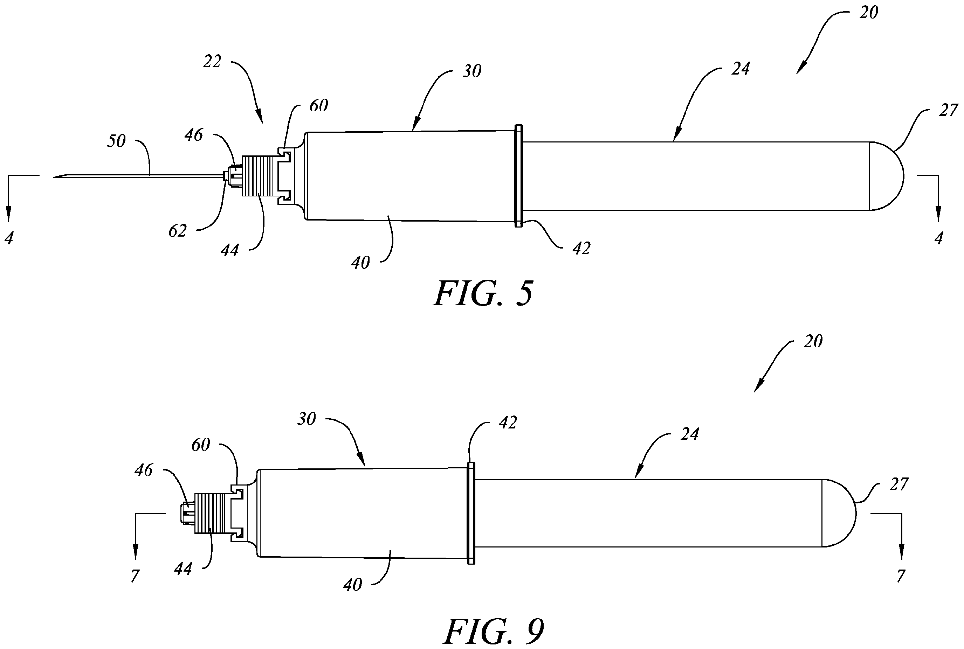

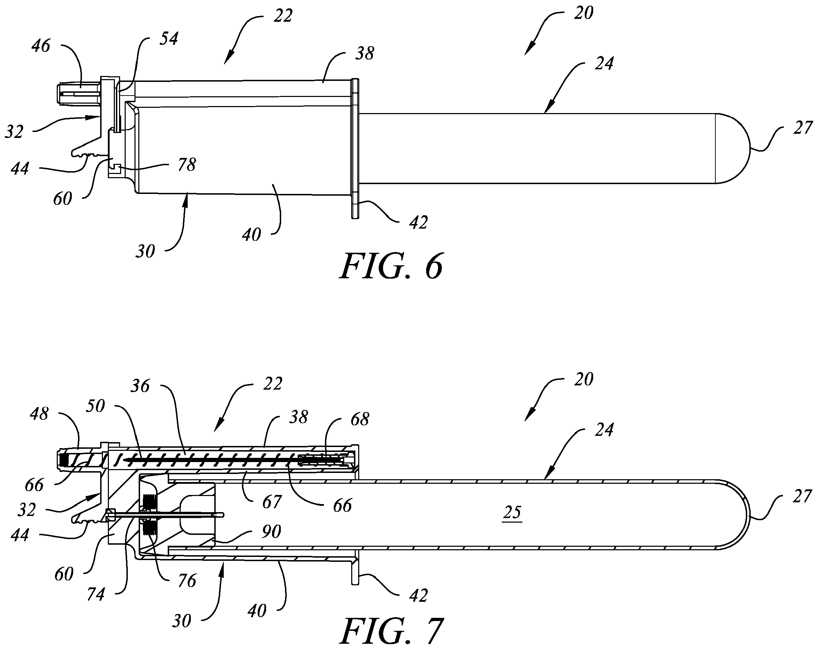

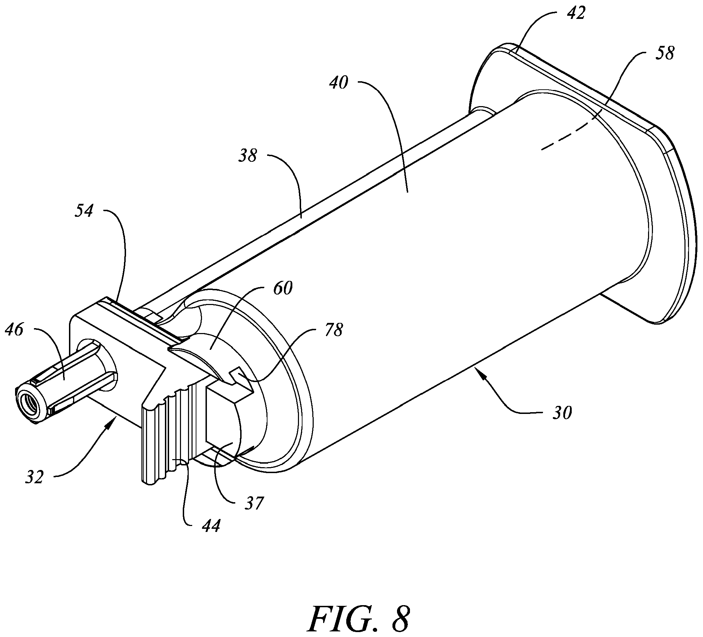

Referring to FIGS. 1-9, a bodily fluid sampling device configured as blood collection tube holder 22 desirably comprises body 30 and frontal attachment 32 that are slidably engaged in such manner that body 30 can slide laterally relative to frontal attachment 32. Body 30 further comprises a substantially cylindrical receptacle 40 having an inside bore 58 with a diameter sufficiently large to receive a fluid collection tube (visible in FIGS. 3-7) through a rearwardly facing opening (not visible) surrounded by flange 42. Needle retraction chamber 38 is laterally spaced apart from bore 58 of body 30 and has one open end and one closed end. Needle retraction chamber 38 also comprises an elongate cylindrical bore 36 that is accessed through forwardly facing opening 34. In this embodiment, substantially cylindrical receptacle 40 and needle retraction chamber 38 are unitarily molded of a suitable polymeric material and share a common wall 67 that is best seen in FIG. 7. It should be understood, however, that the longitudinally extending side walls of receptacle 40 and needle retraction chamber 38 can be laterally spaced apart without a common wall. The front portion of body 30 comprises a substantially cylindrical collar 60 comprising upper and lower slots 78 configured to receive and support top and bottom rails 54, 56 respectively, of frontal attachment 32 to facilitate lateral sliding movement of body 30 relative to frontal attachment 32.

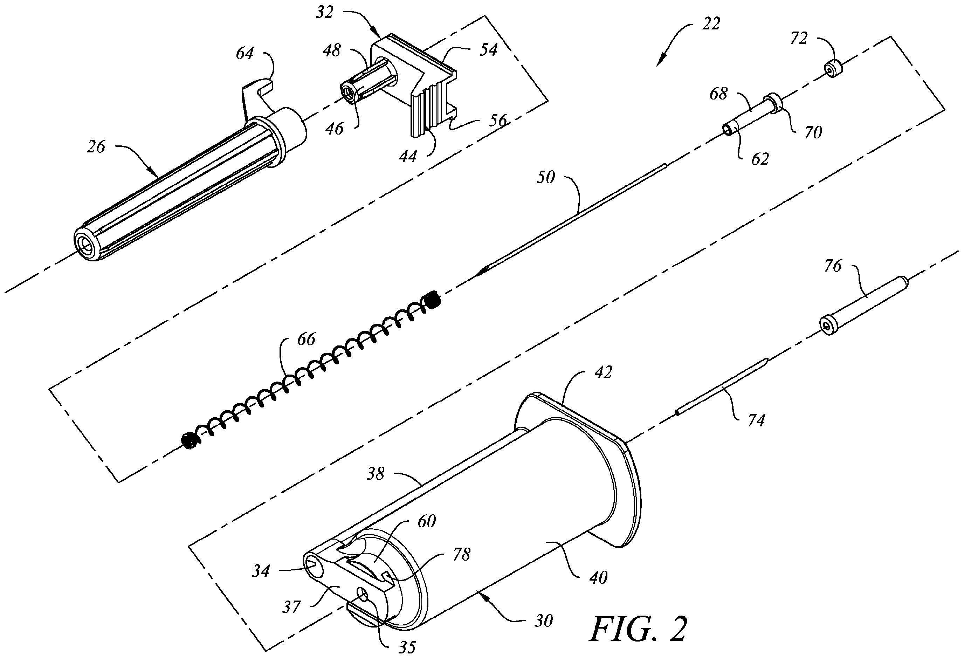

Frontal attachment 32 further comprises digital touchpad 44 facing laterally outward on the side opposite needle retraction chamber. Nose 46 projects forwardly from the slidably engaged portion of frontal attachment 32 and is shown with a plurality of circumferentially spaced, longitudinally extending ribs 48 that provide frictional engagement with a removable needle cap 26 that is shown, for example, in FIGS. 2 and 4. As viewed in FIG. 1, venipuncture needle 50 with beveled needle tip 52 projects forwardly from nose 46. Venipuncture needle 50 is desirably supported by needle holder 68, the forwardly extending tip 62 of which is visible in FIG. 1.

Referring to FIG. 2, additional structural elements are visible that are not apparent from FIG. 1. Removable needle cap 26 further comprises locking arm 64 that projects radially outward and has a rearwardly extending tip that is insertable into opening 34 of body 30 when needle cap 26, frontal attachment 32 and body 30 are all assembled as shown in FIG. 4. When in this position, locking arm 64 prevents body 30 and frontal attachment 32 from shifting relative to each other during shipment and storage prior to use. Still referring to FIG. 2, a needle retraction mechanism comprising needle holder 68 and compression spring 66 is desirably seated inside nose 46 of frontal attachment 32 prior to attachment of frontal attachment 32 to body 40. Compression spring 66 has an inside diameter allowing it to slide over needle 50 and onto the elongate shaft portion of needle holder 68. The needle holder also has a larger diameter head 70 that provides a forwardly facing annular surface that abuts against the rearwardly facing end of compression spring 66 to hold the spring in compression when the forwardly facing end of spring 66 is seated inside nose 46.

Fluid seal 72 is desirably seated inside a cylindrical recess 35 that is laterally spaced apart from opening 34 in front surface 37 of body 30. Front surface 37 cooperates with the rearwardly facing surface (not visible in FIG. 2) of frontal attachment 32 that is disposed between rails 54, 56 to provide another sliding interface between frontal attachment 32 and body 30 that is transverse to venipuncture needle 50 and to fluid discharge needle 74. According to a satisfactory embodiment of the invention, venipuncture needle 50, a longitudinal bore through needle holder 68, an axial bore through fluid seal 72 and fluid discharge needle 74 are all coaxially alignable to establish a continuous fluid flow path through blood collection tube holder 22 during sampling of bodily fluids. The forwardly extending end of fluid discharge needle 74 is desirably seated just behind fluid seal 72 and is covered by flexible sheath 76 at any time that fluid discharge needle 74 is not inserted into and through the stopper of a fluid collection tube. Upon removal of a fluid collection tube from receptacle 40, flexible sheath 76 will again expand and desirably retain any blood or other bodily fluid either trapped or still flowing into tube holder 22 from a patient.

Referring to FIGS. 3 and 4, whenever a bodily fluid collection tube such as blood collection tube 24 (typically having one closed end 27 and one stoppered end 90) is inserted into the opening in the rear of receptacle 40 of body 30, fluid discharge needle 74 contacts and pierces stopper 90, which causes flexible sheath 76 to collapse to the position shown in FIG. 4. After locking needle cap 26 is removed and venipuncture needle 50 is inserted into a patient, blood can flow through needle 50, fluid seal 72 and fluid discharge needle 74 into evacuated reservoir 25 inside blood collection tube 74. Following collection of the sample in tube 24, it can be removed and another tube can be inserted to collect another sample or, if no further fluid samples are needed, body 30 can be shifted from the sample collection position to the needle retraction position without removing venipuncture needle 50 from the patient. For this reason, spring 66 of the needle retraction mechanism is desirably strong enough to retract needle holder 68 and needle 50 into needle retraction cavity prior to withdrawing venipuncture needle 50 from the vascular system of the patient. Although this method of use is preferred to reduce the likelihood of accidental needle sticks and the spread of blood-borne pathogens, venipuncture needle 50 can alternatively be removed from the patient prior to shifting either body 30 or frontal attachment 32 relative to the other along the sliding interface disposed between the two elements to initiate needle retraction.

Movement of body 30 and frontal attachment 32 relative to each other along the transverse sliding interface disposed between them is desirably achieved by applying oppositely directed manual or digital pressure to touchpad 44 of frontal attachment 32 and to the forward portion of the outwardly facing wall of needle retraction chamber 38 of body 30. Where venipuncture needle 50 is still inserted inside a patient, manual pressure is desirably applied to the outside wall of needle retraction chamber 38 while only applying resistance pressure to digital touchpad 44 of frontal attachment 32 so as not to cause venipuncture needle tip 52 to move while inserted in a patient's vein prior to needle retraction.

Referring again to FIGS. 1-9, as body 30 is moved laterally relative to frontal attachment 32 following use of tube holder 22, venipuncture needle 50 is carried by the needle retraction mechanism transversely from a first position where it was coaxially aligned with fluid seal 72 and fluid discharge needle 74 to a second position where it is coaxially aligned with the forwardly facing opening into bore 36 of needle retraction chamber 38. During the transverse movement, the rearwardly extending needle holder head 70 is biased by spring 66 into abutting contact with front surface 37 of body 30. As venipuncture needle 50 and needle holder 68 become coaxially aligned with bore 36 of needle retraction chamber 38, because the diameter of opening 34 is greater than the outside diameter of needle holder head 70 (FIG. 2), spring 66 expands further and drives needle holder 68 and venipuncture needle 50 upwardly into needle retraction chamber 38, thereby preventing needle tip 52 from any longer extending forwardly of nose 46 of frontal attachment 32 and rendering the device "safe" from accidental needle sticks. Similarly, because fluid discharge needle 74 is again covered by expanding flexible sheath 76 upon removal of blood collection tube 24 from receptacle 40, no pathogenically contaminated blood is subject to contact through the opening at the rearwardly facing end of receptacle 40.

Both body 30 and frontal attachment 32 are desirably molded from a moldable polymeric material suitable for the intended use. Fluid seal 72 is desirably made of an elastomeric polymer to facilitate expansion and contraction as needed to prevent leakage during use. If needed, an additional sealing element can be disposed around opening 35 to aid in preventing any fluid leakage during or after use. Venipuncture needle 50 is desirably secured inside needle holder 68 by any known conventional means such as, for example, by the use of glue or another adhesive that cures quickly under ultraviolet light or other radiation. Fluid discharge needle 74 is desirably held in place inside the fluid flow path through body 30 by glue or another adhesive composition, or by frictional engagement with either body 30 or fluid seal 72, or by the use of an additional seating element as discussed in greater detail below in relation to another embodiment of the invention.

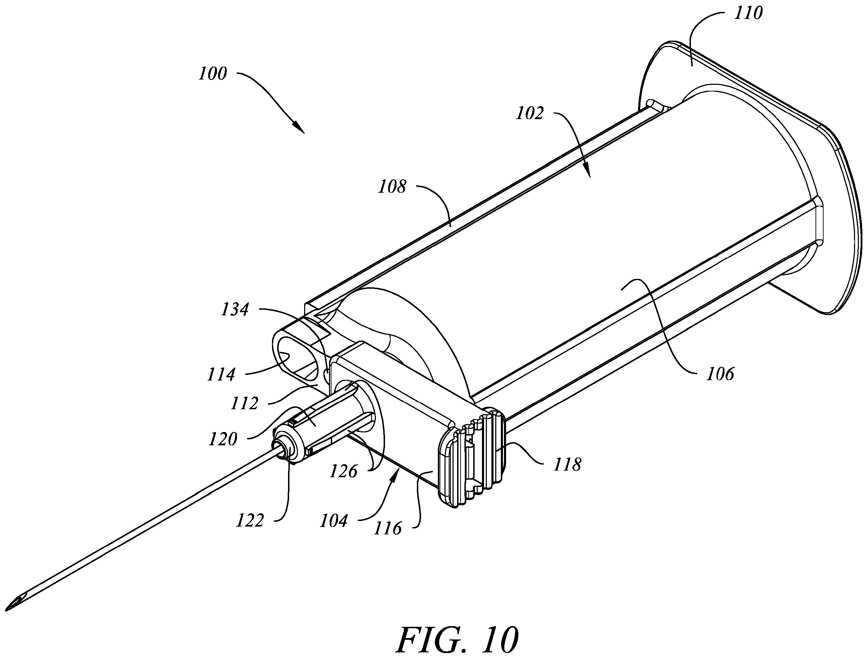

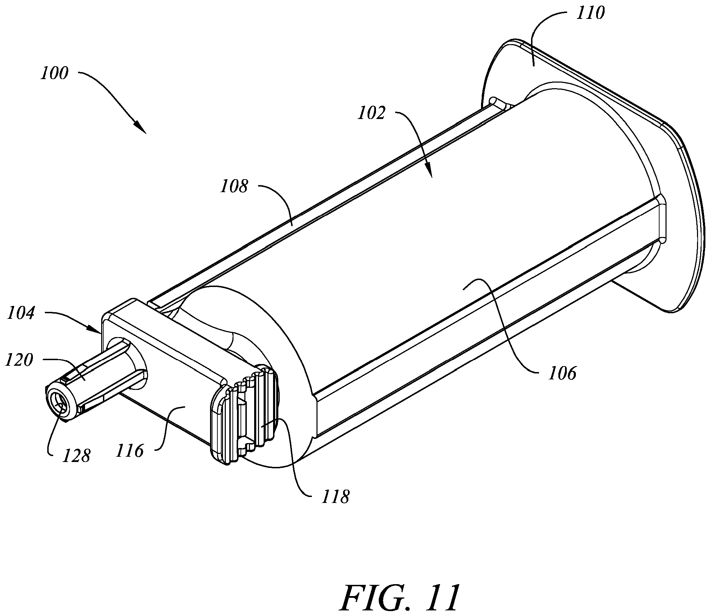

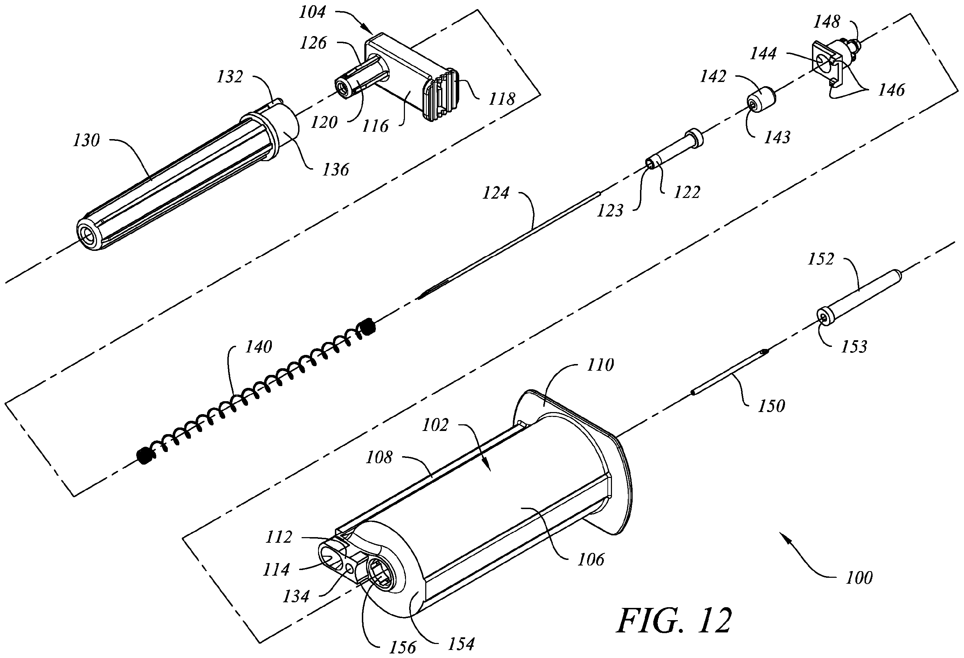

Referring to FIGS. 10-16, a bodily fluid sampling device configured as blood collection tube holder 100 desirably comprises body 102 and frontal attachment 104 that are slidably engaged in such manner that body 102 can slide laterally relative to frontal attachment 104 along at least one sliding interface that is transverse relative to coaxially alignable venipuncture needle 124 and fluid discharge needle 150. Venipuncture needle 124 is held by needle holder 122 having longitudinal bore 123 and projects forwardly from nose 120 of frontal attachment 104 prior to and during use. Locking needle cap 130 (FIGS. 12-14) desirably covers venipuncture needle 124 prior to use, and is releasably secured to frontal attachment 104 by frictional engagement between the inside of attachment collar 136 and the plurality of circumferentially spaced-apart, longitudinally extending ribs 126 disposed around nose 120. In this embodiment, locking arm 132 of locking needle cap 130 is configured and positioned so that it is releasably engageable with a recess defined by an additional forwardly facing, small-diameter opening 134 disposed between oval-shaped opening 114 into needle retraction chamber 108 and larger-diameter opening 156 that is located in front wall 154 of cylindrical receptacle 106 of body 102.

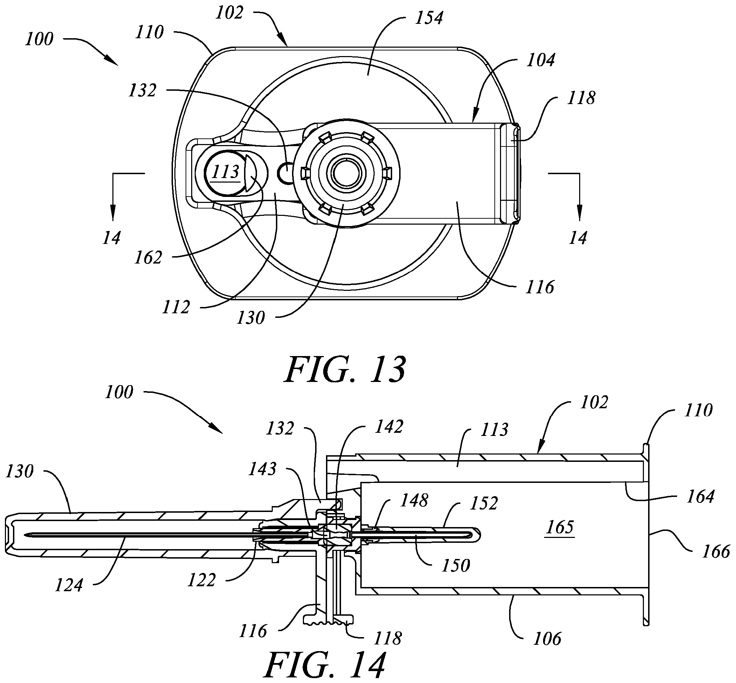

In this embodiment of the invention, a needle retraction mechanism comprising needle holder 122 and compression spring 140 are desirably against seated inside frontal attachment 104 as previously described in relation to the embodiment of FIGS. 1-9, and elastomeric fluid seal 142 containing coaxial fluid passage 143 is against disposed between body 102 and frontal attachment 104. Referring to FIGS. 12, 14, fluid seal 142 is seated in opening 144 inside attachment element 148 that supports and seats fluid discharge needle 150 and base 153 of flexible sheath 152. During assembly of blood collection tube holder 100, attachment element 148 (with fluid discharge needle 150 and flexible sheath 152 already attached) is satisfactorily inserted and seated inside larger-diameter opening 156 by frictional engagement or by the use of a suitable adhesive if desired. Frontal attachment 104 is then attachable to attachment element 148 seated inside body 102 in such manner that upper and lower engagement arms 146 slidably engage at least one laterally extending support rail disposed on the rearwardly facing side of body 116 of frontal attachment 104. Similarly, the back side of body 16 desirably comprises laterally extending sliding surfaces that slidably engage top, bottom or forwardly facing surfaces 112 of body 102 to facilitate transverse slidable engagement of frontal attachment 104 with body 102 along at least one sliding interface disposed between frontal attachment 104 and body 102.

Referring to FIGS. 12-14, forwardly facing oval-shaped opening 114 comprises an inclined ramp section 162 communicating between opening 114 and the remainder of needle retraction cavity 113 inside needle retraction chamber 108. Inclined ramp section 162 serves as guide to begin directing needle holder 122 and spring 140 into needle retraction cavity 113 as body 102 is moved laterally across the face of frontal attachment 104 to a point where needle holder 122 and venipuncture needle 124 are no longer coaxially aligned with fluid seal 142 and fluid discharge needle 150 during the needle retraction process after the desired fluid sample(s) are drawn. It is be noted, however, that FIG. 13 depicts blood collection tube holder 100 before any sample is drawn because locking needle cap 130 is still in place, ramp section 162 is fully visible, and body 102 is not shifted laterally relative to frontal attachment 104 as previously described in relation to the embodiment of FIGS. 1-9.

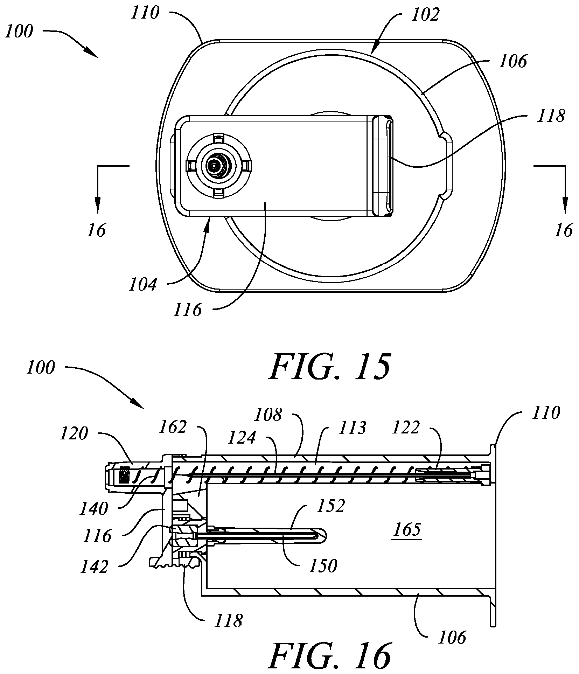

Referring to FIGS. 15-16, body 102 is repositioned relative to frontal attachment 104 by the application of oppositely directed manual pressure against touchpad 118 and the outside wall of needle retraction chamber 113. When repositioned in this manner, which can precede or follow removal of a bodily fluid collection tube (used in sampling bodily fluids such as blood) from interior 165 of cylindrical receptacle 106 through a rearwardly facing opening in flange 110, venipuncture needle 124 is no longer coaxially alignable with fluid discharge needle 150, and the fluid pathway from venipuncture needle 124 through fluid seal 142 and fluid discharge needle 150 is blocked by body 116 of frontal attachment 104. As seen in FIG. 16, spring 140 then expands to force needle holder 122 and at least a major portion of venipuncture needle 124 into needle retraction cavity 113 inside needle retraction chamber 108 so that no portion of needle 124 projects forwardly from nose 120, rendering the device "safe" against accidental needle sticks and the associated risks of pathogenic contamination and the spread of disease.

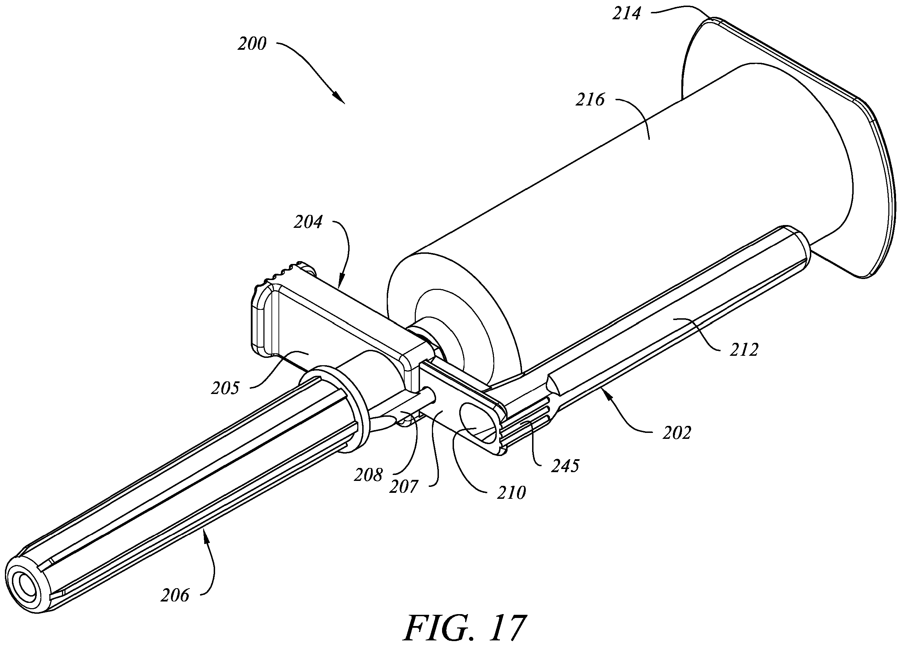

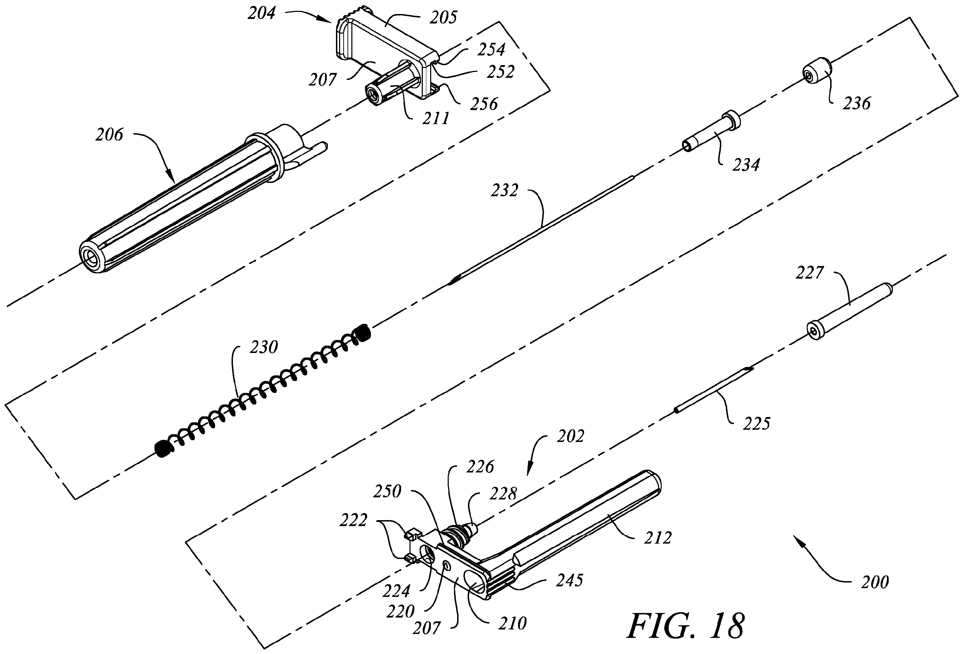

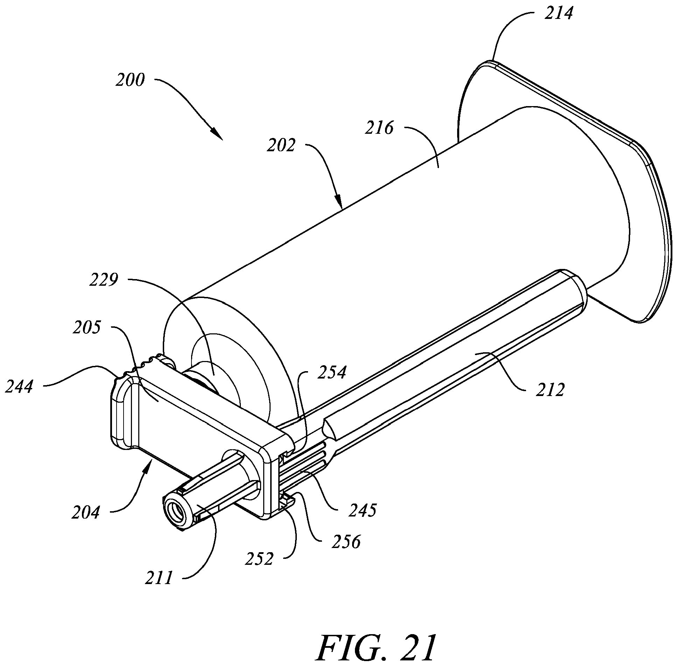

Referring to FIGS. 17-21, a bodily fluid sampling device configured as blood collection tube holder 200 desirably comprises body 202 and frontal attachment 204 that are slidably engageable in such manner that body 202 can slide laterally relative to frontal attachment 204 along at least one sliding interface that is transverse relative to coaxially alignable venipuncture needle 232 and fluid discharge needle 225. In this embodiment of the invention, cylindrical receptacle 216 configured to receive a bodily fluid collection tube (not shown) is made separately from body 202 and is selectively attachable to body 202 by a threaded connector 226 that is integrally molded as part of body 202. Although a threaded connection is shown, it should be understood that other similarly effective connector configuration can also be used within the scope of the invention. Thus, for example and without limitation, a snap connector or bayonet-type connector can also be provided, although the connector portions of body 202 and cylindrical tube receptacle 216 are desirably cooperatively sized and configured. Tip 228 disposed rearwardly of threaded connector 226 is configured to receive and frictionally engage and seat flexible sheath 227 when sheath 227 is placed over fluid discharge needle 225.

Referring to FIGS. 17-19, blood collection tube holder 200 comprises body 202, slidably engageable frontal attachment 204 and locking needle cap 206 with locking arm 208. Frontal attachment 202 further comprises top and forwardly facing surfaces 205, 207, top and bottom rearwardly facing rails 254, 256 defining a recess 252 adapted to slidably engage cooperatively sized and configured rails 250 on the top and bottom of body 202 to facilitate lateral sliding movement of body 202 relative to frontal attachment 204 along a sliding interface that is transverse to the longitudinal axis through forwardly facing venipuncture needle 232 and coaxially alignable, rearwardly facing fluid discharge needle 225. A needle retraction assembly comprising compression spring 230 and needle holder 234 are configured and installed in frontal attachment 204 as previously described. Cylindrical fluid seal 236 comprises a fluid pathway suitable for establishing fluid communication between venipuncture needle 232 and needle holder 234 on the front side and with fluid delivery needle 225 on the back side. Flexible sheath 227, which is pierceable by fluid discharge needle 225, is provided to cover fluid delivery needle 225 when it is not inserted into a fluid collection tube. Forwardly facing openings 210, 220 and 224 in forwardly facing surface 207 of body 202 provide front access to needle retraction chamber 212, to a recess behind opening 220 for locking arm 208, and to fluid discharge needle 232, respectively.

Body 202 further comprises needle retraction chamber 212 having a digital touchpad 245 that is visible on the side facing away from top and bottom engagement arms 222 that cooperate with top and bottom rails 250 to facilitate lateral sliding engagement between body 202 and frontal attachment 204. Repositioning of body 202 relative to frontal attachment 204 is achieved when venipuncture needle 232 is still inserted in a patient by applying digital pressure to touchpad 245 while manually applying oppositely directed resistance to touchpad 244. Alternatively, if venipuncture needle 232 is withdrawn from the patient prior to initiating needle retractions, frontal attachment 204 can be repositioned relative to body 202 by simultaneously applying oppositely directed pressure to both of touchpads 244, 245 to "squeeze" body 202 and frontal attachment in opposite directions along the sliding interface between them until needle holder 234 is aligned with opening 210 to complete retraction of needle 232.

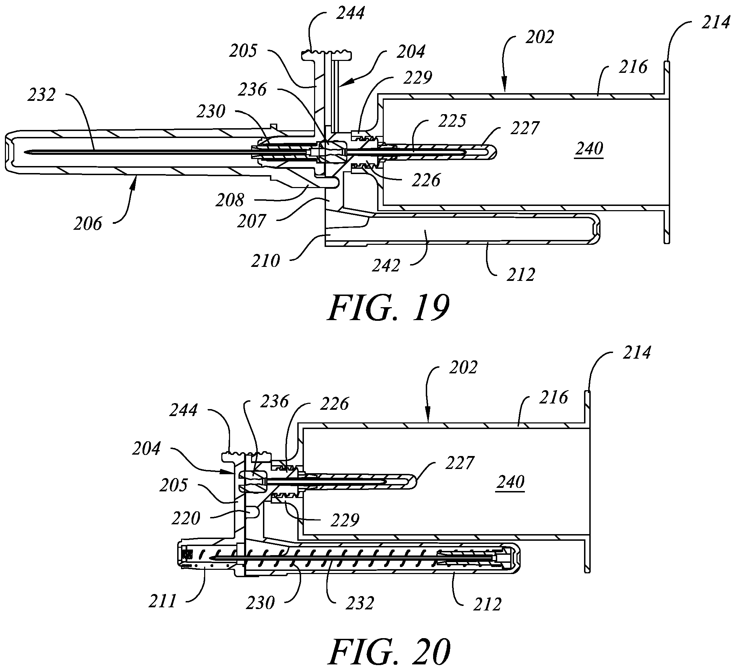

Referring to FIGS. 20-21, cylindrical receptacle 216 comprising an opening defined by rear flange 214 and containing interior volume 240 has a forwardly facing neck 229 comprising female threads configured to engage cooperatively threaded male connector 226 projecting rearwardly from body 202 in laterally spaced-apart relation to needle retraction chamber 212 containing needle retraction cavity 242. Sufficient lateral clearance is desirably provided between threaded connector 226 and needle retraction chamber 212 to permit a cylindrical receptacle 216 of suitable diameter to be positioned between them.

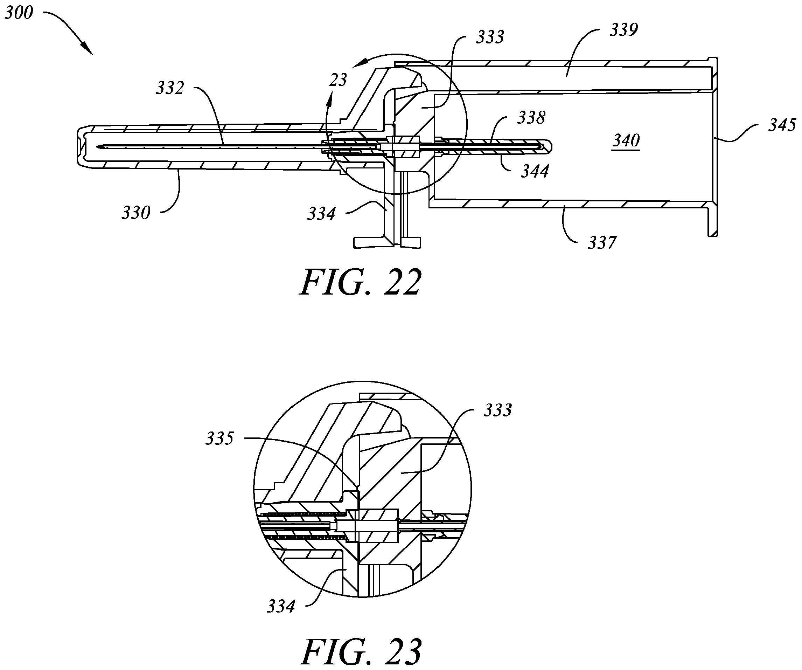

Referring to FIGS. 22 and 23, another embodiment of the invention is disclosed as blood collection tube holder 300 comprising body 333 with cylindrical receptacle 337 having interior volume 340 accessed through open end 345, needle retraction cavity 339, and a rearwardly projecting fluid discharge needle 338 surrounded by flexible sheath 344. Slidably engaging the forwardly facing end of body 333 is frontal attachment 334 comprising forwardly projecting venipuncture needle 332 that is protected in the position shown by locking needle cap 330. Referring to the detail view shown as FIG. 23, an interference element 335 in the configuration of a small projection extending forwardly from body 333 is provided to reduce the likelihood that relative lateral movement will accidentally occur between body 333 and frontal attachment 334 after locking needle cap 330 and its associated locking arm are removed from blood collection tube holder 300 but prior to or during use. Although interference element 335 is depicted as a small projection that can be molded as part of body 333, it will be appreciated upon reading this disclosure that other similarly effective structures such as for example, another rupturable, breakable, frangible, frictional or displaceable physical barrier, can also be used in the invention to reduce the likelihood of accidental or inadvertent, premature lateral movement of the body 333 or frontal attachment 334 relative to the other.

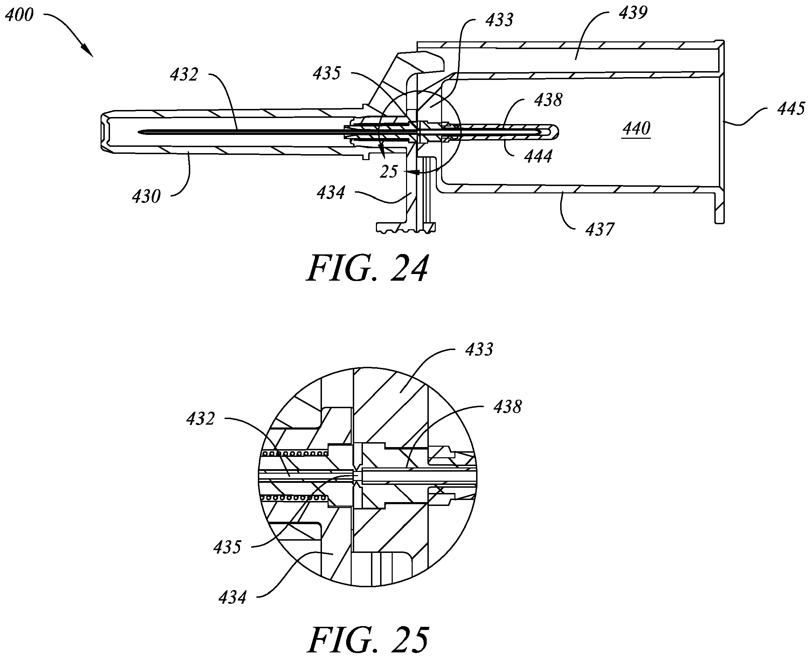

Referring to FIGS. 24 and 25, another embodiment of the invention is disclosed as blood collection tube holder 400 comprising body 433 with cylindrical receptacle 437 having interior volume 440 accessed through open end 445, needle retraction cavity 439, and a rearwardly projecting fluid discharge needle 438 surrounded by flexible sheath 444. Slidably engaging the forwardly facing end of body 433 is frontal attachment 434 comprising forwardly projecting venipuncture needle 432 that is protected in the position shown by locking needle cap 430. Referring to the detail view shown as FIG. 25, in this embodiment of the invention, a small flash chamber 435 is disclosed between the rearwardly facing end of venipuncture needle 432 and the forwardly facing end of fluid discharge needle 438.

Other alterations and modifications of the apparatus disclosed here will become apparent to those of ordinary skill in the art upon reading this specification in relation to the accompanying drawings, and the inventors intend that the scope of the invention be limited only by the broadest interpretation of the appended claims to which the inventors are legally entitled.

## Claims

1. A blood collection tube holder (22, 100, 200, 300, 400) comprising:
a body (30, 102, 202, 333, 433) further comprising a rearwardly facing fluid discharge needle (74, 150, 225, 338, 438) and a needle retraction chamber (38, 108, 113, 212) laterally spaced apart from the fluid discharge needle (74, 150, 225, 338, 438); and
the blood collection tube holder (22, 100, 200, 300, 400) is **characterized by** a frontal attachment (32, 104, 202, 204, 334, 434) disposed in transverse sliding relation to the body (30, 102, 202, 333, 433), the frontal attachment (32, 104, 202, 204, 334, 434) comprising a needle retraction mechanism further comprising a forwardly projecting venipuncture needle (50, 124, 232, 323, 432) that is coaxially alignable with the fluid discharge needle (74, 150, 225, 338, 438) when the frontal attachment (32, 104, 202, 204, 334, 434) is disposed in a first position relative to the body (30, 102, 202, 333, 433), and that is retractable into the needle retraction chamber (38, 108, 113, 212) when the body (30, 102, 202, 333, 433) is repositioned laterally relative to the frontal attachment (32, 104, 202, 204, 334, 434) so that the needle retraction chamber (38, 108, 113, 212) becomes coaxially alignable with the venipuncture needle (50, 124, 232, 323, 432).

2. The blood collection tube holder (22, 100, 200, 300, 400) of claim 1 further comprising a cylindrical receptacle (106, 216, 337, 437) projecting rearwardly from the body (30, 102, 202, 333, 433), the cylindrical receptacle (106, 216, 337, 437) being configured to receive and support a blood collection tube (24) .

3. The blood collection tube holder (400) of claim 1 further comprising a flash chamber (435) disposed between the venipuncture needle (432) and the fluid discharge needle (438).

4. The blood collection tube holder (22, 100, 200, 300, 400) of claim 1 wherein the needle retraction mechanism is a needle retraction assembly seated inside the frontal attachment (32, 104, 202, 204, 334, 434).

5. The blood collection tube holder (22, 100, 200, 300, 400) of claim 4 wherein the needle retraction assembly comprises a needle holder (68, 122, 234) that is rearwardly biased in relation to the frontal attachment (32, 104, 202, 204, 334, 434).

6. The blood collection tube holder (22, 100, 200, 300, 400) of claim 5 wherein the first needle is disposed in fluid communication with the needle holder (68, 122, 234).

7. The blood collection tube holder (22, 100, 200, 300, 400) of claim 1 wherein the fluid discharge needle is seated in fixed relation to the body.

8. The blood collection tube holder (22, 100, 200, 300, 400) of claim 1 wherein the venipuncture needle (50, 124, 232, 323, 432) is selectively retractable relative to the frontal attachment (32, 104, 202, 204, 334, 434).

9. The blood collection tube holder (22, 100, 200, 300, 400) of claim 2 wherein the fluid discharge needle (74, 150, 225, 338, 438) is configured to be insertable into fluid communication with the blood collection tube.

10. The blood collection tube holder (22, 100, 200, 300, 400) of claim 2 wherein the generally cylindrical receptacle (106, 216, 337, 437) is attachable to the body (30, 102, 202, 333, 433).

11. The blood collection tube holder (22, 100, 200, 300, 400) of claim 10 wherein the generally cylindrical receptacle (106, 216, 337, 437) threadedly engages the body (30, 102, 202, 333, 433).

12. The blood collection tube holder (22, 100, 200, 300, 400) of claim 1 wherein the needle retraction chamber (38, 108, 113, 212) has a forwardly facing opening.

13. The blood collection tube holder (22, 100, 200, 300, 400) of claim 2 wherein the needle retraction chamber (38, 108, 113, 212) shares a common wall with the generally cylindrical receptacle (106, 216, 337, 437) and is molded as part of the body (30, 102, 202, 333, 433).

14. The blood collection tube holder (22, 100, 200, 300, 400) of claim 1 wherein the frontal attachment (32, 104, 202, 204, 334, 434) slidably engages the body (30, 102, 202, 333, 433) along a transverse interface that is substantially perpendicular to the fluid discharge needle (74, 150, 225, 338, 438) and extends at least from the fluid discharge needle (74, 150, 225, 338, 438) to the needle retraction chamber (38, 108, 113, 212).

15. The blood collection tube holder (22, 100, 200, 300, 400) of claim 14 wherein relative sliding movement between the frontal attachment (32, 104, 202, 204, 334, 434) and the body (30, 102, 202, 333, 433) can be initiated by applying oppositely directed digital pressure to the frontal attachment (32, 104, 202, 204, 334, 434) and the body (30, 102, 202, 333, 433), respectively.

16. The blood collection tube holder (22, 100, 200, 300, 400) of claim 15 wherein the oppositely directed digital pressure is applied to oppositely facing touchpads disposed on the frontal attachment (32, 104, 202, 204, 334, 434) and the body (30, 102, 202, 333, 433), respectively.

17. The blood collection tube holder (22, 100, 200, 300, 400) of claim 1 further comprising at least one interference element disposed between the frontal attachment (32, 104, 202, 204, 334, 434) and the body (30, 102, 202, 333, 433) to mechanically impede relative sliding movement between the frontal attachment (32, 104, 202, 204, 334, 434) and the body (30, 102, 202, 333, 433) prior to retracting at least part of the first needle into the needle retraction chamber.

18. The blood collection tube holder (22, 100, 200, 300, 400) of claim 17 wherein sliding movement is initiated by manually over-pressuring the interference element.

## Patentansprüche

1. Blutsammelröhrchenhalter (22, 100, 200, 300, 400), umfassend:
einen Körper (30, 102, 202, 333, 433), der ferner eine nach hinten weisende Fluidauslassnadel (74, 150, 225, 338, 438) und eine Nadelrückzugskammer (38, 108, 113, 212) aufweist, die seitlich von der Fluidauslassnadel (74, 150, 225, 338, 438) beabstandet ist; und
der Blutsammelröhrchenhalter (22, 100, 200, 300, 400) **gekennzeichnet ist durch** eine vordere Befestigung (32, 104, 202, 204, 334, 434), die in quer gleitender Beziehung zum Körper (30, 102, 202, 333, 433) angeordnet ist, wobei die vordere Befestigung (32, 104, 202, 204, 334, 434) einen Nadelrückzugsmechanismus umfasst, der ferner eine nach vorne vorstehende Venenpunktionsnadel (50, 124, 232, 323, 432) umfasst, die koaxial mit der Fluidauslassnadel (74, 150, 225, 338, 438) koaxial ausrichtbar ist, wenn die vordere Befestigung (32, 104, 202, 204, 334, 434) in einer ersten Position relativ zum Körper (30, 102, 202, 333, 433) angeordnet ist, und die in die Nadelrückzugskammer (38, 108, 113, 212) zurückziehbar ist, wenn der Körper (30, 102, 202, 333, 433) seitlich relativ zu der vorderen Befestigung (32, 104, 202, 204, 334, 434) verschoben wird, so dass die Nadelrückzugskammer (38, 108, 113, 212) koaxial mit der Venenpunktionsnadel (50, 124, 232, 323, 432) ausrichtbar wird.

2. Der Blutsammelröhrchenhalter (22, 100, 200, 300, 400) nach Anspruch 1 umfasst ferner eine zylindrische Aufnahme (106, 216, 337, 437), die von dem Körper (30, 102, 202, 333, 433) nach hinten vorsteht, wobei die zylindrische Aufnahme (106, 216, 337, 437) so konfiguriert ist, dass sie ein Blutsammelröhrchen (24) aufnimmt und trägt.

3. Der Blutsammelröhrchenhalter (400) nach Anspruch 1 umfasst ferner eine Entspannungskammer (435), die zwischen der Venenpunktionsnadel (432) und der Fluidauslassnadel (438) angeordnet ist.

4. Der Blutsammelröhrchenhalter (22, 100, 200, 300, 400) nach Anspruch 1, wobei der Nadelrückzugsmechanismus eine Nadelrückzugsbaugruppe ist, die innerhalb der vorderen Befestigung (32, 104, 202, 204, 334, 434) sitzt.

5. Der Blutsammelröhrchenhalter (22, 100, 200, 300, 400) nach Anspruch 4, wobei die Nadelrückzugsbaugruppe einen Nadelhalter (68, 122, 234) umfasst, der in Bezug auf die vordere Befestigung (32, 104, 202, 204, 334, 434) nach hinten vorgespannt ist.

6. Der Blutsammelröhrchenhalter (22, 100, 200, 300, 400) nach Anspruch 5, wobei die erste Nadel in Fluidverbindung mit dem Nadelhalter (68, 122, 234) angeordnet ist.

7. Der Blutsammelröhrchenhalter (22, 100, 200, 300, 400) nach Anspruch 1, wobei die Fluidauslassnadel in fester Beziehung zum Körper sitzt.

8. Der Blutsammelröhrchenhalter (22, 100, 200, 300, 400) nach Anspruch 1, bei dem die Venenpunktionsnadel (50, 124, 232, 323, 432) relativ zur vorderen Befestigung (32, 104, 202, 204, 334, 434) selektiv einziehbar ist.

9. Der Blutsammelröhrchenhalter (22, 100, 200, 300, 400) nach Anspruch 2, wobei die Fluidauslassnadel (74, 150, 225, 338, 438) so konfiguriert ist, dass sie in Flüssigkeitsverbindung mit dem Blutsammelröhrchen einführbar ist.

10. Der Blutsammelröhrchenhalter (22, 100, 200, 300, 400) nach Anspruch 2, wobei die allgemein zylindrische Aufnahme (106, 216, 337, 437) an dem Körper (30, 102, 202, 333, 433) befestigbar ist.

11. Der Blutsammelröhrchenhalter (22, 100, 200, 300, 400) nach Anspruch 10, wobei die allgemein zylindrische Aufnahme (106, 216, 337, 437) mit dem Körper (30, 102, 202, 333, 433) in Gewindeeingriff steht.

12. Der Blutsammelröhrchenhalter (22, 100, 200, 300, 400) nach Anspruch 1, wobei die Nadelrückzugskammer (38, 108, 113, 212) eine nach vorne gerichtete Öffnung aufweist.

13. Der Blutsammelröhrchenhalter (22, 100, 200, 300, 400) nach Anspruch 2, wobei die Nadelrückzugskammer (38, 108, 113, 212) eine gemeinsame Wand mit der allgemein zylindrischen Aufnahme (106, 216, 337, 437) teilt und als Teil des Körpers (30, 102, 202, 333, 433) geformt ist.

14. Der Blutsammelröhrchenhalter (22, 100, 200, 300, 400) nach Anspruch 1, wobei die vordere Befestigung (32, 104, 202, 204, 334, 434) gleitend mit dem Körper (30, 102, 202, 333, 433) entlang einer transversalen Grenzfläche in Eingriff steht, die im Wesentlichen senkrecht zur Fluidauslassnadel (74, 150, 225, 338, 438) verläuft und sich mindestens von der Fluidauslassnadel (74, 150, 225, 338, 438) zur Nadelrückzugskammer (38, 108, 113, 212) erstreckt.

15. Der Blutsammelröhrchenhalter (22, 100, 200, 300, 400) nach Anspruch 14, wobei eine relative Gleitbewegung zwischen der vorderen Befestigung (32, 104, 202, 204, 334, 434) und dem Körper (30, 102, 202, 333, 433) durch Aufbringen eines entgegengesetzt gerichteten Fingerdrucks auf die vordere Befestigung (32, 104, 202, 204, 334, 434) bzw. den Körper (30, 102, 202, 333, 433) eingeleitet werden kann.

16. Der Blutsammelröhrchenhalter (22, 100, 200, 300, 400) nach Anspruch 15, wobei der entgegengesetzt gerichtete Fingerdruckauf entgegengesetzt gerichtete Berührungsflächen ausgeübt wird, die an der vorderen Befestigung (32, 104, 202, 204, 334, 434) bzw. am Körper (30, 102, 202, 333, 433) angeordnet sind.

17. Der Blutsammelröhrchenhalter (22, 100, 200, 300, 400) nach Anspruch 1 umfasst ferner mindestens ein Interferenzelement, das zwischen der vorderen Befestigung (32, 104, 202, 204, 334, 434) und dem Körper (30, 102, 202, 333, 433) angeordnet ist, um eine relative Gleitbewegung zwischen der vorderen Befestigung (32, 104, 202, 204, 334, 434) und dem Körper (30, 102, 202, 333, 433) mechanisch zu verhindern, bevor mindestens ein Teil der ersten Nadel in die Nadelrückzugskammer zurückgezogen wird.

18. Der Blutsammelröhrchenhalter (22, 100, 200, 300, 400) nach Anspruch 17, wobei die Gleitbewegung durch manuelles Überdrücken des Interferenzelements eingeleitet wird.

## Revendications

1. Support de tube de collecte de sang (22, 100, 200, 300, 400) comprenant :
un corps (30, 102, 202, 333, 433) comprenant en outre une aiguille d'évacuation de liquide (74, 150, 225, 338, 438) faisant face vers l'arrière et une chambre de rétractation d'aiguille (38, 108, 113, 212) latéralement espacée de l'aiguille d'évacuation de liquide (74, 150, 225, 338, 438) ; et
le support de tube de collecte de sang (22, 100, 200, 300, 400) étant **caractérisé par** une fixation frontale (32, 104, 202, 204, 334, 434) disposée en une relation coulissante transversale par rapport au corps (30, 102, 202, 333, 433), la fixation frontale (32, 104, 202, 204, 334, 434) comprenant un mécanisme de rétractation d'aiguille comprenant en outre une aiguille de ponction veineuse (50, 124, 232, 323, 432) se projetant vers l'avant qui peut être alignée de manière coaxiale sur l'aiguille d'évacuation de liquide (74, 150, 225, 338, 438) lorsque la fixation frontale (32, 104, 202, 204, 334, 434) est disposée dans une première position par rapport au corps (30, 102, 202, 333, 433), et qui est rétractable dans la chambre de rétractation d'aiguille (38, 108, 113, 212) lorsque le corps (30, 102, 202, 333, 433) est repositionné latéralement par rapport à la fixation frontale (32, 104, 202, 204, 334, 434) de sorte que la chambre de rétractation d'aiguille (38, 108, 113, 212) devient coaxialement alignable sur l'aiguille de ponction veineuse (50, 124, 232, 323, 432).

2. Support de tube de collecte de sang (22, 100, 200, 300, 400) selon la revendication 1, comprenant en outre un réceptacle cylindrique (106, 216, 337, 437) se projetant vers l'arrière depuis le corps (30, 102, 202, 333, 433), le réceptacle cylindrique (106, 216, 337, 437) étant conçu pour recevoir et supporter un tube de collecte de sang (24).

3. Support de tube de collecte de sang (400) selon la revendication 1, comprenant en outre une chambre de rinçage (435) disposée entre l'aiguille de ponction veineuse (432) et l'aiguille d'évacuation de liquide (438).

4. Support de tube de collecte de sang (22, 100, 200, 300, 400) selon la revendication 1, le mécanisme de rétractation d'aiguille étant un ensemble de rétractation d'aiguille reposant à l'intérieur de la fixation frontale (32, 104, 202, 204, 334, 434).

5. Support de tube de collecte de sang (22, 100, 200, 300, 400) selon la revendication 4, l'ensemble de rétractation d'aiguille comprenant un support d'aiguille (68, 122, 234) qui est sollicité vers l'arrière en relation avec la fixation frontale (32, 104, 202, 204, 334, 434).

6. Support de tube de collecte de sang (22, 100, 200, 300, 400) selon la revendication 5, la première aiguille étant disposée en communication fluidique avec le support d'aiguille (68, 122, 234).

7. Support de tube de collecte de sang (22, 100, 200, 300, 400) selon la revendication 1, l'aiguille d'évacuation de liquide reposant en relation fixe par rapport au corps.

8. Support de tube de collecte de sang (22, 100, 200, 300, 400) selon la revendication 1, l'aiguille de ponction veineuse (50, 124, 232, 323, 432) étant rétractable de manière sélective par rapport à la fixation frontale (32, 104, 202, 204, 334, 434).

9. Support de tube de collecte de sang (22, 100, 200, 300, 400) selon la revendication 2, l'aiguille d'évacuation de liquide (74, 150, 225, 338, 438) étant conçue pour pouvoir être insérée en communication fluidique avec le tube de collecte de sang.

10. Support de tube de collecte de sang (22, 100, 200, 300, 400) selon la revendication 2, le réceptacle généralement cylindrique (106, 216, 337, 437) pouvant être fixé au corps (30, 102, 202, 333, 433).

11. Support de tube de collecte de sang (22, 100, 200, 300, 400) selon la revendication 10, le réceptacle généralement cylindrique (106, 216, 337, 437) engageant de manière filetée le corps (30, 102, 202, 333, 433).

12. Support de tube de collecte de sang (22, 100, 200, 300, 400) selon la revendication 1, la chambre de rétractation d'aiguille (38, 108, 113, 212) ayant une ouverture faisant face vers l'avant.

13. Support de tube de collecte de sang (22, 100, 200, 300, 400) selon la revendication 2, la chambre de rétractation d'aiguille (38, 108, 113, 212) partageant une paroi commune avec le réceptacle généralement cylindrique (106, 216, 337, 437) et étant moulée comme partie du corps (30, 102, 202, 333, 433).

14. Support de tube de collecte de sang (22, 100, 200, 300, 400) selon la revendication 1, la fixation frontale (32, 104, 202, 204, 334, 434) engageant de manière coulissante le corps (30, 102, 202, 333, 433) le long d'une interface transversale qui est sensiblement perpendiculaire à l'aiguille d'évacuation de liquide (74, 150, 225, 338, 438) et s'étendant au moins depuis l'aiguille d'évacuation de liquide (74, 150, 225, 338, 438) vers la chambre de rétractation d'aiguille (38, 108, 113, 212).

15. Support de tube de collecte de sang (22, 100, 200, 300, 400) selon la revendication 14, le mouvement coulissant relatif entre la fixation frontale (32, 104, 202, 204, 334, 434) et le corps (30, 102, 202, 333, 433) pouvant être initié par l'application d'une pression digitale dirigée à l'opposé par rapport à la fixation frontale (32, 104, 202, 204, 334, 434) et au corps (30, 102, 202, 333, 433), respectivement.

16. Support de tube de collecte de sang (22, 100, 200, 300, 400) selon la revendication 15, la pression digitale dirigée à l'opposé étant appliquée pour faire face de manière opposée à des touches disposées sur la fixation frontale (32, 104, 202, 204, 334, 434) et le corps (30, 102, 202, 333, 433), respectivement.

17. Support de tube de collecte de sang (22, 100, 200, 300, 400) selon la revendication 1, comprenant en outre au moins un élément d'interférence disposé entre la fixation frontale (32, 104, 202, 204, 334, 434) et le corps (30, 102, 202, 333, 433) pour bloquer mécaniquement le mouvement coulissant relatif entre la fixation frontale (32, 104, 202, 204, 334, 434) et le corps (30, 102, 202, 333, 433) avant la rétractation d'au moins une partie de la première aiguille dans la chambre de rétractation d'aiguille.

18. Support de tube de collecte de sang (22, 100, 200, 300, 400) selon la revendication 17, le mouvement coulissant étant initié par la surpression de manière manuelle de l'élément d'interférence.
